# EUROPÄISCHE PATENTSCHRIFT

(11) **EP 0 939 813 B1**
(45) Veröffentlichungstag und Bekanntmachung des Hinweises auf die Patenterteilung: **23.03.2005**
(21) Anmeldenummer: 97940155.1
(22) Anmeldetag: 02.09.1997
(51) Int. Cl.: C12N 15/12, C07K 14/705, C07K 16/30, A61K 38/17, G01N 33/50, C12Q 1/68

(54) **MAMMAKARZINOM-ASSOZIIERTES GEN**
BREAST CARCINOMA-ASSOCIATED GENE
GENE ASSOCIE AU CARCINOME MAMMAIRE

(30) Priorität: 03.09.1996 EP 96114098
(43) Veröffentlichungstag der Anmeldung: 08.09.1999
(73) Patentinhaber: Roche Diagnostics GmbH, 68305 Mannheim (DE)
(72) Erfinder: WEIDLE, Ulrich, D-80336 München (DE); SCHIEMANN, Sabine, D-67657 Kaiserslautern (DE)
(86) Internationale Anmeldenummer: PCT/EP1997/004785
(87) Internationale Veröffentlichungsnummer: WO 1998/010065

(56) Entgegenhaltungen:
- WO-A-97/19171
- MARVIN KW ET AL: "Identification and characterization of a novel squamous cell-associated gene related to PMP22" JOURNAL OF BIOLOGICAL CHEMISTRY, Bd. 270, Nr. 48, 1.Dezember 1995, MD US, Seiten 28910-28916, XP000616413 in der Anmeldung erwähnt
- RUEGG CL ET AL.: "B4B, a novel growth-arrest gene, is expressed by a subset of progenitor/pre-B lymphocytes negative for cytoplasmic mu-chain" JOURNAL OF IMMUNOLOGY, Bd. 157, Nr. 1, 1.Juli 1996, BALTIMORE US, Seiten 72-80, XP000616417
- SCHIEMANN, SABINE ET AL: "Differential gene expression in human mammary carcinoma cells: identification of a new member of a receptor family" ANTICANCER RES., 1997, 17, 13-20, XP002049575

## Beschreibung

Die vorliegende Erfindung betrifft eine pharmazeutische Zusammensetzung, ihre Verwendung zur Tumordiagnostik, Therapie und Prävention, Verfahren zur Diagnose, Therapie und Prävention von Tumoren, sowie Antikörper und ihre Verwendung.

Das unregulierte Wachstum von Tumorzellen wird durch ein neues Muster der Expression von Genen hervorgerufen, welche die Zellzykluskontrolle, Adhäsion, Angiogenese, Invasivität und schließlich die Metastasenbildung steuern (Pardee, Advances in Cancer Res. 65 (1994), 213-227; Ponta et al., Biochem. Biophys. Acta 1198 (1994), 1-10). Der klinische Verlauf von Tumorerkrankungen, wie etwa Brustkrebs, ist durch mehrere definierte molekulare Vorgänge charakterisiert, wie etwa Östrogen-unabhängiges Wachstum, Tamoxifen-Resistenz, Expression von Vimentin, Erhöhung der Invasivität und schließlich Kreuzresistenz gegenüber einer großen Anzahl von chemotherapeutischen Mitteln, die häufig als Multi Drug-Resistenz bezeichnet wird (vgl. z. B. Clarke et al., J. Endocrinol. 122 (1989), 331-340; Sommers et al., Cancer Res. 53 (1992), 5190-5197; Sommers et al., Cancer Res. 49 (1989), 4258-4263 und Saceda et al., Mol. Endocrinol. 2 (1988), 1157-1162).

Es ist ein zentrales Anliegen der Tumorforschung, Gene zu identifizieren, die am Entstehen und am Fortschreiten von Tumorerkrankungen wesentlich beteiligt sind und auf Basis dieser Gene neue Mittel zur Tumordiagnose, -prävention oder -therapie bereitzustellen.

Die vorliegende Erfindung beschreibt die Identifizierung, Klonierung und Charakterisierung des Gens für ein Polypeptid, das als Progressions-assoziiertes Protein "PAP" bezeichnet wird. Dieses Protein wird in der metastasierenden humanen Mammakarzinom-Zellinie MCF-7_{ADR} exprimiert, während in der nicht metastasierenden Mammakarzinom-Zellinie MCF-7 keine Expression gefunden wurde. Das PAP-Protein, eine dafür codierende Nucleinsäure sowie gegen das Protein gerichtete Antikörper sind somit als Mittel zur Diagnostik, Therapie oder Prävention von Tumorerkrankungen, insbesondere von Brustkrebs, geeignet.

Durch die Erfindung wird eine pharmazeutische Zusammensetzung bereitgestellt, die dadurch gekennzeichnet ist, daß sie als aktive Komponente enthält:
(A) eine Nucleinsäure, die
   (a) in SEQ ID No. 1 dargestellte Protein-codierende Nucleotidsequenz,
   (b) eine der Sequenz aus (a) im Rahmen der Degeneration des genetischen Codes entsprechende Nucleotidsequenz,
   (c) eine mit den Sequenzen aus (a) oder/und (b) unter stringenten Bedingungen hybridisierende Sequenz oder
   (d) einen mindestens 20 Nucleotide langen Abschnitt, der Sequenzen aus (a), (b) oder/und (c) umfaßt,
(B) ein Polypeptid oder Peptid, das von einer Nucleinsäure gemäß (A) kodiert ist, oder/und
(C) einen Antikörper gegen ein Polypeptid oder Peptid gemäß (B).

Unter stringenten Hybridisierungsbedingungen ist in diesem Zusammenhang zu verstehen, daß eine Hybridisierung auch nach Waschen bei 55°C, vorzugsweise bei 62°C, besonders bevorzugt bei 68°C in einem Niedrigsalz-Puffer (z.B. 0,2 x SSC, 0,1% SCS) noch auftritt (siehe auch Sambrock et al., (1989), Molecular Cloning, A Laboratory Manual, Cold Spring Harbor Laboratory Press).

In einer bevorzugten Ausführungsform enthält die pharmazeutische Zusammensetzung weiterhin pharmazeutisch übliche Träger-, Hilfs- oder Zusatzstoffe.

Die in SEQ ID No.1 dargestellte Protein-codierende Nucleotidsequenz codiert für ein Protein mit einer Länge von 157 Aminosäuren, welche der von Ruegg et al., 1996 (B4B, a Novel Growth-Arrest Gene, Is Expressed by a Subset of Progenitor/Pre-B Lymphocytes Negative for Cytoplasmic µ-Chain, Am. Ass. Imm., 1996, S. 72-80) beschriebenen B4B-Nucleotidsequenz entspricht und eine Homologie zu bereits bekannten Proteinen aufweist. In den von Ruegg et al. (supra) durchgeführten Versuchen soll die Expression des B4B-Proteins einen Wachstumsarrest in Cos-7-Zellen bewirken. Deshalb wird die Verwendung des B4B-Proteins als potentieller Tumorsuppressor postuliert.

Eines dieser Proteine ist das Kaninchen-Protein CL-20, das während der Differenzierung von Kaninchentrachealzellen in vitro induziert wird und am häufigsten in Plattenepithelgeweben vorkommt (Marvin, J. Biol. Chem. 270 (1995), 28910-28916). Die Expression von CL-20 ist nicht von den Wachstumsbedingungen abhängig, aber Retinoide, welche die Plattenepitheldifferenzierung inhibieren, reprimieren die Induktion von CL-20.

Ein weiteres, zu PAP homologes Protein ist das Ratten-Epithelialmembranprotein EMP-1, welches hauptsächlich in den Proliferations- und Differenzierungszonen der äußeren Magendrüse sowie in Epithelzellen der Magenregion vorkommt (Taylor et al., J. Biol. Chem. 270 (1995), 28824-28833).

Außerdem ist PAP homolog zum peripheren Myelinprotein PMP22 aus Mensch, Maus und Ratte, bei dem es sich um ein Myelinassoziiertes transmembranes Strukturprotein handelt. PMP22 ist ein für die Wachstumshemmung spezifisches Protein, das die Zellzyklus-Progression verhindert (Hayasaka et al., BBRC 186 (1992), 827-831; Edomi et al., Gene 126 (1993), 289-290; Welcher et al., Proc. Natl. Acad. Sci. USA 88 (1991), 7195-7198; Spreyer et al., EMBO J. 10 (1991), 3661-3668).

Aufgrund der Daten von Ruegg et al. und der Homologie zu dem für das Aufrechterhalten des zellulären Ruhezustands verantwortlichen PMP22-Protein war es im höchsten Ausmaß überraschend, daß das PAP-Protein selektiv in der stark entarteten metastasierenden Zellinie MCF-7_{ADR}, aber nicht in der weniger entarteten Zellinie MCF-7 exprimiert wird.

Ferner weist PAP eine hohe Homologie zu bisher noch nicht publizierten Proteinen auf, deren Sequenzen in der Genbank gespeichert sind. Diese Proteine werden als murines TMP bzw. humanes TMP bezeichnet und haben eine Aminosäure-Identität von 76 % bzw. 95 % zu PAP.

Die Nucleotid- und Aminosäuresequenzen von murinem TMP sind in SEQ ID No.3 und 4 und von humanem TMP in SEQ ID No.5 und 6 dargestellt. Die Unterschiede zwischen PAP und humanem TMP liegen insbesondere im Bereich der Aminosäuren 32-48.

Weiterhin ein Gegenstand der vorliegenden Erfindung ist die Verwendung eines Vektors, der mindestens eine Kopie einer Nucleinsäure wie zuvor definiert oder einen Abschnitt davon enthält zur Transformation einer Zelle. Diese Nucleinsäure kann z. B. genomische DNA, cDNA oder mRNA sein. Vorzugsweise handelt es sich um ein rekombinantes DNA-Molekül.

Die Erfindung betrifft auch die Verwendung eines Vektors, der einen mindestens 20 Nucleotide langen Abschnitt der in SEQ ID No.1 dargestellten Protein-codierenden Sequenz enthält. Vorzugsweise besitzt dieser Abschnitt eine für PAP-spezifische Nucleotidsequenz. Diese Nucleinsäuren eignen sich insbesondere zur Herstellung von therapeutisch einsetzbaren Antisense-Nucleinsäuren, die vorzugsweise bis zu 50 Nucleotide lang sind.

Der die Nucleinsäure enthaltende Vektor kann in Eukaryonten oder Prokaryonten replizierbar sein. Er kann ein in das Genom der Wirtszelle integrierbarer Vektor, z. B. Bakteriophage λ, oder ein Vektor sein, der extrachromosomal vorliegt (z. B. ein Plasmid). Der erfindungsgemäße Vektor kann durch Subklonierung der PAP-DNA in einen Basisvektor erhalten werden. Derartige Basisvektoren, insbesondere Vektoren mit den zur Proteinexpression erforderlichen Elementen sind einem Fachmann geläufig.

Bei Klonierung einer für PAP-codierenden Nucleinsäure kann ein Expressionsvektor hergestellt werden, der in einer geeigneten Wirtszelle zur Expression gebracht wird, wobei das erfindungsgemäße Protein entsteht. Bevorzugte Wirtszellen sind Mikroorganismen, wie E.coli oder Hefe, aber auch höhere Zellen, z. B. Säuger- oder Insektenzellen. Bevorzugte Expressionsvektoren sind z. B. Plasmide, Bakteriophage λ für Prokaryonten, Hefevektoren oder virale Vektoren für höhere Zellen, z. B. SV40, Vaccinia, Baculovirus. Bezüglich der Expression einer PAP-codierten Nucleinsäure soll insbesondere auf die bei Sambrook et al. (supra) genannten Methoden verwiesen werden.

Zur Expression von PAP geeignete Systeme enthalten geeignete Vektoren, z. B. den Vektor pcDNA1 (Invitrogen), bei dem die zu exprimierende DNA sich unter Kontrolle des Cytomegaloviruspromotors befindet.

Ein weiterer Gegenstand der vorliegenden Erfindung ist eine Zelle, die dadurch gekennzeichnet ist, daß sie mit einer Nucleinsäure transformiert ist, die
(a) die in SEQ ID No. 1 dargestellte Protein kodierende Nucleotidsequenz,
(b) eine der Sequenz aus (a) im Rahmen der Degeneration des genetischen Codes entsprechende Nucleotidsequenz oder
(c) eine Nucleotidsequenz, die eine Homologie von mehr als 95% zu einer Sequenz aus (a) oder/und (b) umfaßt, mit der Maßgabe, daß die Zelle keine Cos-7-Zelle ist.

Die Zelle kann sowohl eine eukaryontische als auch eine prokaryontische Zelle sein. Verfahren zur Transformation von Zellen mit Nucleinsäuren sind allgemeiner Stand der Technik und brauchen daher nicht näher erläutert werden.

Ebenfalls ein Gegenstand der vorliegenden Erfindung ist die Verwendung eines PAP-Polypeptids, mit der in SEQ ID No. 2 dargestellten Amminosäuresequenz, Fragmenten dieses Polypeptids oder Varianten davon als Immunogen zur Herstellung von Antikörpern.

Unter Varianten sind Sequenzen zu verstehen, die sich durch Substitution, Deletion oder/und Insertion einzelner Aminosäuren oder kurzer Aminosäureabschnitte von der in SEQ ID No. 2 dargestellten Aminosäuresequenz unterscheiden.

Unter den Begriff "Variante" fallen sowohl natürlich vorkommende allelische Variationen von PAP sowie durch rekombinante DNA-Technologie (insbesondere durch in vitro-Mutagenese mit Hilfe von chemisch-synthetisierten Oligonucleotiden) erzeugte Proteine, die hinsichtlich ihrer biologischen oder/und immunologischen Aktivität dem in SEQ ID No.2 dargestellten Protein im wesentlichen entsprechen.

In einer bevorzugten Ausführungsform betrifft die Erfindung eine Verwendung der pharmazeutischen Zusammensetzung zur Tumordiagnostik, besonders bevorzugt zur Diagnostik von Mammakarzinomen. Besonders bevorzugt ist weiter eine Verwendung als Tumorprogressionsmarker, dessen Expression mit dem Fortschreiten des Tumors oder/und mit dem Metastasierungsgrad korreliert.

In einer besonders bevorzugten Ausführungsform zur Diagnose von Tumoren korreliert die Expressionsstärke des PAP-Proteins mit dem Tumorstadium, insbesondere mit der Neigung zur Metastasenbildung.

Noch ein weiterer Gegenstand der vorliegenden Erfindung ist ein Verfahren zur Diagnose von Tumoren, wobei man ein aus einem Patienten stammendes Gewebe mit einer erfindungsgemäßen pharmazeutischen Zusammensetzung in Kontakt bringt und die Expression des PAP-Proteins qualitativ oder/und quantitativ bestimmt. Diese Bestimmung kann beispielsweise auf Nucleinsäureebene durch Verwendung von Nucleinsäure-Hybridisierungssonden oder über reverse Transkription/PCR bzw. auf Proteinebene durch Antikörper nach cyto- oder histochemischen Methoden erfolgen. Besonders bevorzugt ist die Verwendung der pharmazeutischen Zusammensetzung als Tumorprogressionsmarker zur Identifizierung der Aggressivität von Tumoren, insbesondere Mammakarzinomen, durch Quantifizierung der PAP-Expression, beispielsweise nach einem operativen Eingriff oder zur Verlaufskontrolle bei einer chemotherapeutischen Behandlung.

Neben Nucleinsäuren und Polypeptiden von PAP sind auch Antikörper gegen Polypeptide oder Fragmente davon als Bestandteile der pharmazeutischen Zusammensetzung geeignet.

Besonders bevorzugt sind polyklonale Antikörper gegen ein wie oben beschriebenes Polypeptid, mit der Maßgabe, daß der Antikörper nicht gegen die Peptidsequenz CSDSLSYASEDALK oder CSHY-ANRDGTOQYHH gerichtet ist oder ein monoklonaler Antikörper gegen ein Polypeptid wie oben beschrieben. Am meisten bevorzugt ist ein Antikörper, der dadurch gekennzeichnet ist, daß er gegen eine Peptidsequenz gerichtet ist, die den Aminosäuren 32 bis 48, 49 bis 62 oder 119 bis 129 der in SEQ ID No. 2 dargestellten Aminosäuresequenz entspricht.

Die Herstellung von Antikörpern kann dabei auf übliche Weise durch Immunisierung von Versuchstieren mit dem vollständigen PAP-Protein oder Fragmenten davon und anschließende Gewinnung der resultierenden polyklonalen Antiseren erfolgen. Nach der Methode von Köhler und Milstein oder deren Weiterentwicklungen können aus den Antikörper-produzierenden Zellen der Versuchstiere auf bekannte Weise durch Zellfusion monoklonale Antikörper erhalten werden. Ebenso können nach bekannten Methoden humane monoklonale Antikörper hergestellt werden.

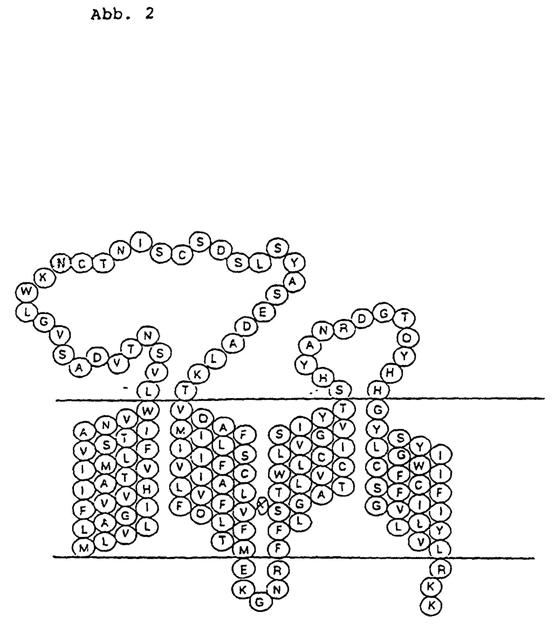

Als Immunogen bevorzugt sind Peptide, die aus den extrazellulären Domänen von PAP (vgl. Fig. 2) stammen. Besonders bevorzugte Peptide stammen aus Bereichen, die den Aminosäuren 32-48, 49-62 oder 119-129 von SEQ ID No.2 entsprechen. Diese Peptide werden vorzugsweise nach bekannten Methoden an einen Träger, z. B. Keyhole Limpet Hemocyanin nach bekannten Methoden (Snipes et al., J. Cell. Biol. 117 (1992), 225-238) gekoppelt. Die resultierenden Konjugate werden zur Immunisierung von Versuchstieren, z. B. Kaninchen, verwendet.

Ein weiterer Gegenstand der vorliegenden Erfindung ist somit ein Antikörper gegen das PAP-Protein oder eine Variante davon, vorzugsweise ein Antikörper, der keine Kreuzreaktion mit homologen Proteinen wie EMP-1, PMP22 und CL-20 zeigt. Besonders bevorzugt ist der Antikörper gegen eine Peptidsequenz gerichtet, die den Aminosäuren 32 bis 48, 49 bis 62 oder 119 bis 129 der in SEQ ID No.2 dargestellten Aminosäuresequenz entspricht.

Ein weiterer Gegenstand der Erfindung ist die Verwendung der oben beschriebenen Antikörper in einem immunologischen Verfahren einer Immunpräzipitation, eines Western-Blots, eines kompetetiven Immuntests oder eines Sandwichtests.

Noch ein weiterer Gegenstand der Erfindung betrifft die Verwendung eines Polypeptids oder eines Fragments davon, das kodiert wird durch
(a) die in SEQ ID No. 1 dargestellte Protein kodierende Nucleotidsequenz,
(b) eine der Sequenz aus (a) im Rahmen der Degeneration des genetischen Codes entsprechende Nucleotidsequenz oder
(c) eine Nucleotidsequenz, die eine Homologie von mehr als 95% zu einer Sequenz aus (a) oder/und (b) umfaßt, zur Herstellung eines Antikörpers unter Verwendung einer Phage-Display-Antikörperbibliothek.

Mit diesem Verfahren wird die Identifizierung und Herstellung eines Antikörpers gegen das PAP-Protein ausschließlich mit Methoden der rekombinanten DNA-Technologie ohne die Verwendung von Tieren oder aus Tieren gewonnenen (primären) Zellen möglich.

Die bei einer "klassischen" Antikörperherstellung nötigen arbeits- und zeitaufwendigen Schritte wie Immunisierung von Versuchstieren, Boosten oder Zellfusion und Selektionszyklen von Einzelklonen entfallen somit.

Die vorgestellten Ergebnisse schaffen überdies die Voraussetzung für eine gezielte Diagnostik von Krankheiten, die mit Veränderungen der PAP-Aktivität ursächlich verbunden sind. Diese Untersuchungen können mit Hilfe spezifischer Nucleinsäuresonden zum Nachweis auf Nucleinsäureebene, d.h. auf Gen- bzw. Transkriptebene oder mit Hilfe von Antikörpern gegen PAP zum Nachweis auf Proteinebene durchgeführt werden.

Die Erfindung wird durch die folgenden Beispiele, Abbildungen und Sequenzprotokolle näher erläutert. Es zeigen:
- Abb. 1: einen Vergleich der Aminosäuresequenzen zwischen humanem PAP (1), humanem (2) und Maus (3) TMP, Ratten EMP-1 (4), Kaninchen CL-20 (5), humanem (6), Maus (7) und Ratten (8) PMP 22,
- Abb. 2: eine Vorhersage der Topologie von PAP in einer Lipiddoppelschicht. Der gefüllte Kreis bedeutet eine potentielle N-Glykosilierungsstelle,
- SEQ ID No.1: eine Nucleinsäuresequenz, welche die für PAP-codierende genetische Information enthält,
- SEQ ID No.2: die Aminosäuresequenz von PAP,
- SEQ ID No.3: die Nucleinsäuresequenz von Maus TMP cDNA,
- SEQ ID No.4: die Aminosäuresequenz von Maus TMP,
- SEQ ID No.5: die Nucleinsäuresequenz von humaner TMP cDNA und
- SEQ ID No.6: die Aminosäuresequenz von humanem TMP.

### Beispiele

### Beispiel 1

### Kultivierung von Zellinien

Die Zellinien MCF-7 und MCF-7_{ADR} sind humane Mammakarzinom-Zellinien (Thompson et al., J. Cell. Physiol. 150 (1992), 534-544 und Fairchild et al., Cancer Res. 47 (1987), 5141-5148.

Die Zellinie MCF-7 ist nicht-metastasierend, exprimiert den Östrogen-Rezeptor, zeigt Östrogen-abhängiges Wachstum in der Nacktmaus und keine Expression von Vimentin. Die Zellinie MCF-7_{ADR} leitet sich von MCF-7 ab und wurde über ihre Resistenz gegen Adriamycin selektiert. Sie ist metastasierend, zeigt eine Multi Drug-Resistenz, keine Expression des Östrogen-Rezeptors sowie Östrogen-unabhängiges Wachstum in der Nacktmaus.

Die Kultivierung dieser Zellinien erfolgte in Improved Minimal Essential Medium (IMEM; Gibco, Grand Island, NY) mit 5 % fötalem Rinderserum (Gibco). Die Zellen wurden bei 37 °C und einer 5 % CO₂-Atmosphäre kultiviert.

### Beispiel 2

### Differential-Display PCR

Die Differential-Display-Polymerase-Kettenreaktion (DD-PCR) wurde nach bekannten Methoden (vgl. Liang und Pardee, Science 275 (1992), 967-970; Liang et al., Cancer Res. 52 (1992), 6966-6968 und Liang et al., Nucleic Acids Res. 21 (1993), 3269-3275) unter Verwendung des RNA-map-Kits (GenHunter Corp., Brookline, MA) gemäß den Vorschriften des Herstellers durchgeführt.

Aus MCF-7 und MCF-7_{ADR}-Zellen wurde Gesamt-RNA nach der Methode von Chomczynski und Sacchi (Anal. Biochem. 162 (1987), 156-159) unter Verwendung des Total-RNA Isolation System (Promega Corp., Madison, WI) isoliert. Die Beseitigung von DNA-Kontaminationen aus den RNA-Proben erfolgte durch Inkubation mit RNase freier DNase I unter Verwendung des Message-Clean-Kit (GenHunter Corp., Brookline, MA) durch Inkubation für 30 min bei 37 °C.

DNA-freie Gesamt-RNA (0,2 µg) aus MCF-7 und MCF-7_{ADR}-Zellen wurde als Matrize für die cDNA-Erststrangsynthese in Gegenwart von 10 µM der Ankerprimer T₁₂MG, T₁₂MC, T₁₂MA und T₁₂MT (wobei M dreifach degeneriert für G, A und C ist), 1 X Reverse Transkriptase-Puffer (125 mM Tris-Cl, pH 8,3; 188 mM KCl; 7,5 mM MgCl₂; 25 mM Dithiothreitol) und 250 µM dNTP-Mischung verwendet. Die Lösung wurde 5 min. auf 65 °C erhitzt, für 10 min auf 37 °C abgekühlt und dann wurden 200 U Moloney murine Leukemiavirus (MMLV) Reverse Transkriptase zugegeben. Nach einstündiger Inkubation bei 37 °C wurde die Reaktion durch 5-minütige Inkubation bei 95 °C gestoppt.

Die PCR wurde in einer Reaktionslösung durchgeführt, die 0,1 Volumina des Ansatzes zur Reversen Transkription, 10 µM des jeweiligen T₁₂MN-Ankerprimer, 2 µM 10-mer Primer mit willkürlich festgelegter Sequenz, 1 x-PCR-Puffer (100 mM Tris-Cl, pH 8,4; 500 mM KCl; 15 mM MgCl₂; 0,01 % Gelatine), 25 µM dNTP, 10 µCi [α-³⁵S] dATP und 10 U Amplitaq DNA-Polymerase (Perkin Elmer, Norwalk, CT) enthielt. Die PCR wurde für insgesamt 40 Zyklen bei 94 °C für 30 s, 40 °C für 2 min, 72 °C für 30 s und schließlich 5 min bei 72 °C durchgeführt.

Nach Zugabe von Beladungspuffer zu jeweils 3,5 µl Probe wurden die PCR-Produkte 2 min lang bei 80 °C erhitzt und dann auf ein denaturierendes 5 % Polyacrylamid-Sequenziergel zur Elektrophorese aufgetragen. Das getrocknete Gel wurde autoradio-graphisch auf differentiell exprimierte Gene analysiert.

Die in zwei unabhängigen DD-PCR-Reaktionen identifizierten Banden, die differentiell exprimierten Genen entsprechen, wurden aus dem getrockneten Gel ausgeschnitten. Die cDNA wurde aus dem Gel durch Einweichen des Gelstücks in 100 µl H₂O für 10 min und anschließendes Kochen für 15 min eluiert. Die cDNA wurde durch Ethanolfällung in Gegenwart von 3 M Natriumacetat und 50 µg Glycogen als Träger isoliert und in 10 µl H₂O aufgenommen. 4 µl der eluierten cDNA wurden in einer zweiten PCR reamplifiziert. Dabei wurden dieselben 5'- und 3'-Primer und die oben beschriebenen Bedingungen verwendet, außer daß dNTP-Konzentrationen von 20 µM verwendet wurden und das Reaktionsgemisch keine Radioisotopen enthielt.

Die auf diese Weise erhaltenen amplifizierten PCR-Fragmente wurden über ein 1,5 % Agarosegel aufgetrennt, gereinigt und als Sonden für die Northern-Analyse vcn RNA aus MCF-7 und MCF-7_{ADR} verwendet.

### Beispiel 3

### Northern Analyse

PolyA⁺RNA wurde aus Gesamt-RNA unter Verwendung des PolyA T-tractIII mRNA-Isolierungssystem (Promega Corp., Madison, WI) isoliert. Parallele Spuren von PolyA⁺ RNA aus MCF-7 und MCF-7_{ADR}-Zellen (1 µg von jeder Zellinie) wurden auf einem denaturierenden 1%-Agarose-Formaldehyd-Gel nach Größe aufgetrennt und dann auf eine positiv geladene Nylonmembran (Boehringer Mannheim GmbH, Mannheim) durch Kapillarblotting in 20 x SSC überführt. Nach UV-Quervernetzung (Stratagene UV-Stratalinker 1800) wurden die Membranen mit [α-³²P]dCTP-markierten DD-PCR-Produkten hybridisiert, die mit der "random-primed" DNA-Markierungsmethode hergestellt und bis zu einer spezifischen Aktivität von 2 x 10⁸ dpm/µg unter Verwendung des Rediprime DNA-Markierungssystems (Amersham, Braunschweig) markiert worden waren. Die Vorhybridisierung (5 h) und Hybridisierung (über Nacht) mit radioaktiven Sonden erfolgte in 50% Formamid, 5% SSC, 5 x Denhardt-Lösung, 1% SDS und 100 µg/ml denaturierter Lachssperma-DNA bei 42°C. Die Membranen wurden mit 1 x SSC, 0,1% SDS bei Raumtemperatur für 15 min. 2 x gewaschen, gefolgt von Waschen mit 0,25 x SSC, 0,1% SDS bei 55 - 60°C für 15 bis 30 min. Dann wurden die Membranen autoradiographisch untersucht.

Diejenigen DD-PCR-Produkte, bei denen eine differentielle mRNA-Expression nachgewiesen werden konnte, wurden durch das TA-Cloningsystem (Invitrogen, San Diego, CA) in den PCR II-Vektor subkloniert. Die subklonierten Fragmente wurden unter Verwendung des Quiagen-Plasmidkits (Quiagen, Hilden) isoliert und erneut als Sonden für die Northern Analyse zur Verifizierung der differentiellen mRNA-Expression verwendet.

### Beispiel 4

### Charakterisierung von DD-PCR-Fragmenten, die differentiell exprimierten mRNAs entsprechen

Die subklonierten Fragmente, welche sich von differentiell exprimierten mRNAs ableiten, wurden im TA-Klonierungsvektor unter Verwendung des Dye-Terminator Cycle Sequencing kit (Applied Biosystems GmbH, Foster City, CA) sequenziert. Die Nukleotidsequenzdaten wurden auf Homologie mit bekannten Genen in den Datenbanken Genbank und EMBL unter Verwendung des Computerprogramms BLAST analysiert.

Auf diese Weise konnten neben 10 bekannten mRNA-Spezies eine neue mRNA identifiziert werden, die in der Zellinie MCF-7ADR differentiell exprimiert wird.

Um die vollständige cDNA dieser neuen mRNA zu erhalten, wurde ein 556 bp langes subkloniertes DD-PCR-Fragment als Sonde zur Musterung einer humanen Herz cDNA-Bank (Clontech, Palo Alto, CA) verwendet, die in einem Lambda gt10-Vektor hergestellt worden war. Auf diese Weise wurde ein etwa 2 kb cDNA-Klon isoliert, von dem beide Stränge sequenziert und mit dem klonierten DD-PCR-Fragment verglichen wurden.

Zur Identifizierung der 5'-Region der cDNA wurde eine RACE (Rapid Amplification of cDNA ends) PCR nach der von Frohmann (PCR-Meth. Appl. 4 (1994), 40-58) und Schaefer (Anal. Biochem. 227 (1995), 255-273) beschriebenen Methode mit den Modifikationen von Kato et al. (Gene 150 (1994), 243-250) durchgeführt. Das auf diese Weise erhaltene 1 kb lange 5' RACE PCR-Produkt wurde sequenziert und mit dem aus der Musterung der cDNA-Bank erhaltenen cDNA Klon verglichen.

Die resultierende Nukleotidsequenz der vollständigen cDNA wurde mit bekannten DNA-Sequenzen aus der Genbank und der EMBL-Datenbank verglichen.

Wie durch Northern Blot-Analyse festgestellt, wird die neu identifizierte mRNA ausschließlich in der Zellinie MCF-7_{ADR} exprimiert. Daher wurden die cDNA und das dafür codierende Protein als "Progressions-assoziiertes Protein" (PAP) bezeichnet. Die Nucleotid- und Aminosäuresequenzen von PAP sind in SEQ ID No.1 und 2 angegeben. Die Nucleotidsequenz umfaßt 2786 nt mit einem offenen Leserahmen von 471 nt (157 Aminosäuren), der mit einem ATG-Initiationscodon bei Position 219 beginnt und mit einem TAA-Stopcodon an Position 692 endet. Ein potentielles N-Glycosilierungssignal im Bereich der Aminosäuren 43-45 wurde mit Aminosäure 43 als möglichem Akzeptor der Zuckergruppe identifiziert. Eine potentielle Acylierungsstelle durch kovalente Addition von Myristat wurde an Aminosäureposition 39 identifiziert. Eine mögliche Phosphorylierungsstelle für die Serin-Threonin-Kinase Caseinkinase II wurde an Position 48 identifiziert.

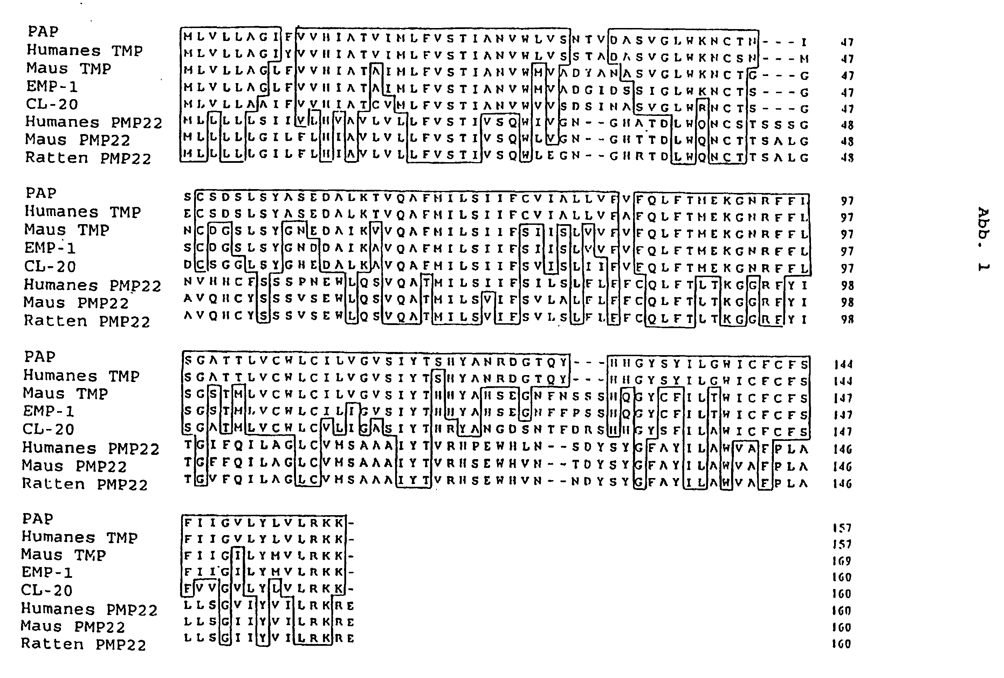

Die PAP cDNA ist Mitglied einer Genfamilie. Ein Vergleich der Aminosäuresequenzen von Mitgliedern dieser Genfamilie ist in Fig. 1 dargestellt. Die höchste Ähnlichkeit hat das humane PAP zum Kaninchen-Protein CL-20 und zum Ratten-Protein EMP-1 (Identität 77 bzw. 76 %) gefolgt vom Protein PMP-22 aus Mensch, Maus und Ratte (Identität von 41 %, 43 % und 41 %).

Weiterhin wurde festgestellt, daß PAP eine große Ähnlichkeit mit bislang nicht publizierten Proteinen hat, die als murines Tumor-assoziiertes Membranprotein (Maus TMP, Genbank U25633, SEQ ID NO. 3/4) und humanes Tumor-assoziiertes Membran-protein, Genbank U43916, SEQ ID NO. 5/6) bezeichnet werden. Murines TMP hat eine Länge von 160 Aminosäuren, wobei 39 Aminosäuren unterschiedlich zu PAP sind (Identität 76%). Humanes TMP hat eine Länge von 157 Aminosäuren, wobei 7 Aminosäuren unterschiedlich zu PAP sind (Identität 95%).

Basierend auf computerunterstützte Hydrophobizitätsplots und Vorhersagen der Sekundärstruktur kann ein Modell für die hypothetische Topologie von PAP vorgeschlagen werden (Fig. 2).

Demzufolge enthält PAP 4 hydrophobe potentielle Transmembrandomänen und 2 potentielle extrazelluläre Domänen (Aminosäuren 29-63 bzw. 118-130).

Im Bereich der ersten extrazellulären Domäne, insbesondere im Bereich der Aminosäuren 32-48, liegen die größten Unterschiede von PAP zu humanem TMP (5 Aminosäuren).

### Beispiel 5

### Expressionsmuster von PAP

Um die gewebespezifische Expression von PAP zu untersuchen, wurde die Verteilung von PAP-mRNA in verschiedenen humanen Geweben durch Northern Blot-Analyse unter Verwendung von Multiple Tissue Northern Blots (Clontech, Palo Alto, CA) untersucht.

Dabei wurde festgestellt, daß PAP-mRNA in den meisten humanen Geweben exprimiert wird. PAP-mRNA fehlt jedoch in peripheren Blutleukozyten und wird nur sehr schwach im Gehirn exprimiert. Weiterhin fehlte PAP-mRNA in verschiedenen Leukämie- und Lymphom-Zellinien wie etwa HL 60 (Promyeolozytäre Leukämie), K562 (chronische myeoloische Leukämie), Molt-4 (lympho-blastoide Leukämie), Raji (Burkitt's Lymphom) und HeLa (Zervikal-Karzinom). Eine starke Expression von PAP-mRNA wurde jedoch in SW480-Zellen (Kolorektales Adenokarzinom) und in G361-Melanomzellen festgestellt..

### Beispiel 6

### Herstellung eines PAP-Expressionskonstrukts und transiente Expression in COS-Zellen

Die PAP-cDNA wurde in die EcoRV-Stelle des Expressionsvektors pcDNA1 (Invitrogen, San Diego, CA) stromabwärts vom Cytomegalovirus-Promotor subkloniert. Der Stammvektor ohne cDNA-Insertion wurde als negative Kontrolle verwendet. Rekombinante DNA wurde über eine Quiagensäule (Quiagen, Hilton, DE) gereinigt und durch Bestimmung der Absorption bei 260 nm quantifiziert.

COS-Zellen, die in DMEM mit 10 % fötalem Kälberserum exponentiell wuchsen, wurden mit Trypsin behandelt, mit Phosphat-gepufferter Salzlösung gewaschen und bei 800 g zentrifugiert. 1,5 x 10⁶-Zellen wurden in 200 µl Phosphat-gepufferter Salzlösung mit 5 µg Vektor DNA resuspendiert. Die Zellen wurden 5 min auf Eis gekühlt, in eine Elektro-porationsküvette (Biorad, Herkules, CA) mit einem Spalt von 4 mm überführt und bei 300 V und 125 Mikrofarad einer Elektroporation unterzogen.

Die transfizierten Zellen wurden für 5 min auf Eis gekühlt und auf sieben 35 mm Kulturschalen mit 2 ml DMEM und 10 % fötalem Kälberserum aufgeteilt und für 48 h kultiviert. Dann wurden die Zellen mit Tris-gepufferter Salzlösung gewaschen, in DMEM mit 2 % Paraformaldehyd für 30 min fixiert, mit Tris-gepufferter Salzlösung gewaschen und 30 min in Tris-gepufferter Salzlösung mit 0,1 % Saponin permeabilisiert. Unspezifische Bindungsstellen wurden 30 min bei Raumtemperatur in Tris-gepufferter Salzlösung, 2 % Rinderserumalbumin, 1 % Schweinehautgelatine (Typ A Sigma), 2 % Ziegenserum und 0,1 % Saponin bloKkiert. Die Zellen wurden mit Anti-PAP-Antikörpern (Beispiel 7), 1 : 500 in Blockierungspuffer mit 0,02 % Saponin verdünnt, über Nacht bei 4 °C inkubiert. Dann wurde Fluoresceinisothiocyanat-markiertes Ziegen-Anti-Kaninchen-Immunglobulin, 1 : 200 in Blockierungspuffer mit 0,02 % Saponin verdünnt, zugegeben. Nach Waschen mit Tris-gepufferter Salzlösung wurden die Zellen auf Objektträger aufgebracht und die Immunreaktivität durch konfokale Mikroskopie mit einem Biorad MRC-600-Scanner zusammen mit einem Zeiss Axiophot Fluoreszenzmikroskop sichtbar gemacht. Als negative Kontrolle wurde Präimmunserum (1 : 500) als primäres Antiserum verwendet.

### Beispiel 7

### Antikörperherstellung

Anti-Peptid-Antikörper wurden gegen synthetische Peptide aus den ersten und zweiten extrazellulären Loops von PAP hergestellt:

Die Peptide wurden an Keyhole-Limpet-Hemocyanin nach bekannten Methoden (Snipes et al., (1992), Supra) gekoppelt. Die Konjugate wurden zur Immunisierung von Kaninchen mit komplettem Freund'schem Adjuvans verwendet. Anschließend folgte eine Auffrischung der Immunisierung in zweiwöchigen Intervallen für insgesamt 4 mal mit 500 µg Peptid und inkomplettem Freund'schem Adjuvans. Von den immunisierten Tieren wurde Blut entnommen und das Serum isoliert. Die Aktivität des Immunserums wurde durch Festphasen-ELISA (Beispiel 6) getestet.

## Patentansprüche

1. Verwendung einer Zusammensetzung, umfassend als aktive Komponente
(A) eine Nukleinsäure, die die
(a) in SEQ ID No. 1 dargestellte Protein-codierende Nucleotidsequenz,
(b) eine der Sequenz aus (a) im Rahmen der Degeneration des genetischen Codes entsprechende Nucleotidsequenz,
(c) eine mit den Sequenzen aus (a) oder (b) unter stringenten Bedingungen hybridisierende Sequenz oder
(d) einen mindestens 20 Nucleotide langen Abschnitt der Sequenzen aus (a), (b) oder (c) umfasst,
(B) ein Progressions-assoziiertes Polypeptid oder Peptid ("PAP"), das von einer Nukleinsäure gemäß (A) codiert ist, oder
(C) einen Antikörper gegen ein Polypeptid oder Peptid gemäß (B), zur Herstellung eines Mittels für die Diagnostik von Mammakarzinomen, kolorektalen Adenokarzinomen oder Melanomen, wobei die Expression des PAP-Polypeptids oder Peptids mit dem Tumorstadium korreliert.

2. Verwendung nach Anspruch 1 zur Diagnostik von Mammakarzinomen.

3. Verwendung nach Anspruch 1 als Tumorprogressionsmarker.

4. Verwendung nach einem der Ansprüche 1 bis 3,
**dadurch gekennzeichnet,**
**dass** man für die Diagnostik ein aus dem Patienten stammendes Gewebe mit einer Zusammensetzung nach Anspruch 1 oder 2 in Kontakt bringt und die Expression des PAP-Polypeptids oder Peptids qualitativ oder/und quantitativ bestimmt.

5. Verwendung nach Anspruch 4,
**dadurch gekennzeichnet,**
**dass** die Expressionsstärke des PAP-Polypeptids oder Peptids mit der Metastasenbildung in Geweben korreliert.

6. Verwendung nach einem der Ansprüche 1 bis 5,
**dadurch gekennzeichnet,**
**dass** der Antikörper ein monoklonaler Antikörper ist.

7. Verwendung eines Antikörpers nach Anspruch 6
**dadurch gekennzeichnet,**
**dass** er gegen eine Peptidsequenz gerichtet ist, die den Aminosäuren 32-48, 49-62 oder 119-129 der in SEQ ID No. 2 dargestellten Aminosäuresequenz entspricht.

## Claims

1. Use of a composition comprising as active components
(A) a nucleic acid which comprises
(a) the protein-coding nucleotide sequence shown in SEQ ID NO.1,
(b) a nucleotide sequence corresponding to the sequence from (a) within the scope of the degeneracy of the genetic code,
(c) a sequence hybridizing with the sequences from (a) or (b) under stringent conditions or
(d) a section of the sequences from (a), (b) or (c) which is at least 20 nucleotides long,
(B) a progression-associated polypeptide or peptide ("PAP") which is coded by a nucleic acid according to (A) or
(C) an antibody against a polypeptide or peptide according to (B) to produce an agent for diagnosing mammary carcinomas, colorectal adenocarcinomas or melanomas where the expression of the PAP polypeptide or peptide correlates with the stage of the tumour.

2. Use as claimed in claim 1 to diagnose mammary carcinomas.

3. Use as claimed in claim 1 as a tumour progression marker.

4. Use as claimed in one of the claims 1 to 3,
**characterized in that**
for the diagnosis a tissue derived from the patient is contacted with a composition as claimed in claim 1 or 2 and the expression of the PAP polypeptide or peptide is determined qualitatively or/and quantitatively.

5. Use as claimed in claim 4,
**characterized in that**
the expression level of the PAP polypeptide or peptide is correlated with the formation of metastases in tissues.

6. Use as claimed in one of the claims 1 to 5,
**characterized in that**
the antibody is a monoclonal antibody.

7. Use of an antibody as claimed in claim 6,
**characterized in that**
it is directed against a peptide sequence which corresponds to the amino acids 32-48, 49-62 or 119-129 of the amino acid sequence shown in SEQ ID NO.2

## Revendications

1. Utilisation d'une composition comprenant en tant que composant actif
(A) un acide nucléique qui comprend
(a) la séquence nucléotidique codant pour la protéine représentée dans SEQ ID n° 1,
(b) une séquence nucléotidique correspondant à la séquence de (a) dans le cadre de la dégénérescence du code génétique,
(c) une séquence s'hybridant dans des conditions stringentes avec les séquences de (a) ou (b) ou
(d) un segment d'une longueur d'au moins 20 nucléotides des séquences de (a), (b) ou (c),
(B) un peptide ou polypeptide associé à la progression ("PAP"), qui est codé par un acide nucléique selon (A),
ou
(C) un anticorps dirigé contre un peptide ou polypeptide selon (B),
pour la préparation d'un agent pour le diagnostic de carcinomes mammaires, d'adénocarcinomes colorectaux ou de mélanomes, l'expression du peptide ou polypeptide PAP étant en corrélation avec le stade tumoral.

2. Utilisation selon la revendication 1, pour le diagnostic de carcinomes mammaires.

3. Utilisation selon la revendication 1, en tant que marqueur de la progression tumorale.

4. Utilisation selon l'une quelconque des revendications 1 à 3, **caractérisée en ce que** pour le diagnostic on met un tissu provenant du patient en contact avec une composition selon la revendication 1 ou 2 et on détermine qualitativement et/ou quantitativement l'expression du peptide ou polypeptide PAP.

5. Utilisation selon la revendication 4, **caractérisée en ce que** l'intensité d'expression du peptide ou polypeptide PAP est en corrélation avec la formation de métastases dans des tissus.

6. Utilisation selon l'une quelconque des revendications 1 à 5, **caractérisée en ce que** l'anticorps est un anticorps monoclonal.

7. Utilisation d'un anticorps selon la revendication 6, **caractérisée en ce qu'**il est dirigé contre une séquence peptidique qui correspond aux aminoacides 32-48, 49-62 ou 119-129 de la séquence aminoacide représentée dans SEQ ID n° 2.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Strasse
      (C) ORT: Mannheim-Waldhof
      (E) LAND: Deutschland
      (F) POSTLEITZAHL: 68305
   (ii) BEZEICHNUNG DER ERFINDUNG: Neues Mammakarzinom-asso ziiertes Gen
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 2786 Basenpaare
      (B) ART: Nucleotid
      (C) STRANGFORM: beides
      (D) TOPOLOGIE: linear
   (ii) ART DES MOLEKÜLS: cDNA
   (vii) UNMITTELBARE HERKUNFT:
      (B) CLON(E): Human PAP
   (ix) MERKMAL:
      (A) NAME/SCHLÜSSEL: CDS
      (B) LAGE:219..689
   (xi) SEQUENZBESCHREIBUNG: SEQ ID NO: 1:
   AGCACTCTCC AGCCTCTCAC CGCAAAATTA CACACCCCAG TACACCAGCA GAGGAAACTT      60
   ATAACCTCGG GAGGCGGGTC CTTCCCCTCA GTGCGGTCAC ATACTTCCAG AAGAGCGGAC     120
   CAGGGCTGCT GCCAGCACCT GCCACTCAGA GCGCCTCTGT CGCTGGGACC CTTCAGAACT     180
   CTCTTTGCTC ACAAGTTACC AAAAAAAAAA GAGCCAAC ATG TTG GTA TTG CTG         233
                                            Met Leu Val Leu Leu
                                             1               5
   GCT GGT ATC TTT GTG GTC CAC ATC GCT ACT GTT ATT ATG CTA TTT GTT       281
   Ala Gly Ile Phe Val Val His Ile Ala Thr Val Ile Met Leu Phe Val
                   10                  15                  20
   AGC ACC ATT GCC AAT GTC TGG TTG GTT TCC AAT ACG GTA GAT GCA TCA       329
   Ser Thr Ile Ala Asn Val Trp Leu Val Ser Asn Thr Val Asp Ala Ser
               25                  30                  35
   GTA GGT CTT TGG AAA AAC TGT ACC AAC ATT AGC TGC AGT GAC AGC CTG       377
   Val Gly Leu Trp Lys Asn Cys Thr Asn Ile Ser Cys Ser Asp Ser Leu
           40                  45                  50
   TCA TAT GCC AGT GAA GAT GCC CTC AAG ACA GTG CAG GCC TTC ATG ATT       425
   Ser Tyr Ala Ser Glu Asp Ala Leu Lys Thr Val Gln Ala Phe Met Ile
       55                  60                  65
   CTC TCT ATC ATC TTC TGT GTC ATT GCC CTC CTG GTC TTC GTG TTC CAG       473
   Leu Ser Ile Ile Phe Cys Val Ile Ala Leu Leu Val Phe Val Phe Gln
    70                  75                  80                  85
   CTC TTC ACC ATG GAG AAG GGA AAC CGG TTC TTC CTC TCA GGG GCC ACC       521
   Leu Phe Thr Met Glu Lys Gly Asn Arg Phe Phe Leu Ser Gly Ala Thr
                   90                  95                 100
   ACA CTG GTG TGC TGG CTG TGC ATT CTT GTG GGG GTG TCC ATC TAC ACT 569
   Thr Leu Val Cys Trp Leu Cys Ile Leu Val Gly Val Ser Ile Tyr Thr
   105 110 115
   AGT CAT TAT GCG AAT CGT GAT GGA ACG CAG TAT CAC CAC GGC TAT TCC 617
   Ser His Tyr Ala Asn Arg Asp Gly Thr Gln Tyr His His Gly Tyr Ser
   120 125 130
   TAC ATC CTG GGC TGG ATC TGC TTC TGC TTC AGC TTC ATC ATC GGC GTT 665
   Tyr Ile Leu Gly Trp Ile Cys Phe Cys Phe Ser Phe Ile Ile Gly Val
   135 140 145
   CTC TAT CTG GTC CTG AGA AAG AAA TAAGGCCGGA CGAGTTCATG GGGATCTGGG 719
   Leu Tyr Leu Val Leu Arg Lys Lys
   150 155
   GGGTGGGGAG GAGGAAGCCG TTGAATCTGG GAGGGAAGTG GAGGTTGCTG TACAGGAAAA 779
   ACCGAGATAG GGGAGGGGGG AGGGGGAAGC AAAGGGGGGA GGTCAAATCC CAAACCATTA 839
   CTGAGGGGAT TCTCTACTGC CAAGCCCCTG CCCTGGGGAG AAAGTAGTTG GCTAGTACTT 899
   TGATGCTCCC TTGATGGGGT CCAGAGAGCC TCCCTGCAGC CACCAGACTT GGCCTCCAGC 959
   TGTTCTTAGT GACACACACT GTCTGGGGCC CCATCAGCTG CCACAACACC AGCCCCACTT 1019
   CTGGGTCATG CACTGAGGTC CACAGACCTA CTGCACTGAG TTAAAATAGC GGTACAAGTT 1079
   CTGGCAAGAG CAGATACTGT CTTTGTGCTG AATACGCTAA GCCTGGAAGC CATCCTGCCC 1139
   TTCTGACCCA AAGCAAAACA TCACATTCCA GTCTGAAGTG CCTACTGGGG GGCTTTGGCC 1199
   TGTGAGCCAT TGTCCCTCTT TGGAACAGAT ATTTAGCTCT GTGGAATTCA GTGACAAAAT 1259
   GGGAGGAGGA AAGAGAGTTT GTAAGGTCAT GCTGGTGGGT TAGCTAAACC AAGAAGGAGA 1319
   CCTTTTCACA ATGGAAAACC TGGGGGATGG TCAGAGCCCA GTCGAGACCT CACACACGGC 1379
   TGTCCCTCAT GGAGACCTCA TGCCATGGTC TTTGCTAGGC CTCTTGCTGA AAGCCAAGGC 1439
   AGCTCTTCTG GAGTTTCTCT AAAGTCACTA GTGAACAATT CGGTGGTAAA AGTACCACAC 1499
   AAACTATGGG ATCCAAGGGG CAGTCTTGCA ACAGTGCCAT GTTAGGGTTA TGTTTTTAGG 1559
   ATTCCCCTCA ATGCAGTCAG TGTTTCTTTT AAGTATACAA CAGGAGAGAG ATGGACATGG 1619
   CTCATTGTAG CACAATCCTA TTACTCTTCC TCTAACATTT TTGAGGAAGT TTTGTCTAAT 1679
   TATCAATATT GAGGATCAGG GCTCCTAGGC TCAGTGGTAG CTCTGGCTTA GACACCACCT 1739
   GGAGTGATCA CCTCTTGGGG ACCCTGCCTA TCCCACTTCA CAGGTGAGGC ATGGCAATTC 1799
   TGGAAGCTGA TTAAAACACA CATAAACCAA AACCAAACAA CAGGCCCTTG GGTGAAAGGT 1859
   GCTATATAAT TGTGAAGTAT TAAGCCTACC GTATTTCAGC CATGATAAGA ACAGAGTGCC 1919
   TGCATTCCCA GGAAAATACG AAAATCCCAT GAGATAAATA AAAATATAGG TGATGGGCAG 1979
   ATCTTTTCTT TAAAATAAAA AAGCAAAAAC TCTTGTGGTA CCTAGTCAGA TGGTAGACGA 2039
   GCTGTCTGCT GCCGCAGGAG CACCTCTATA CAGGACTTAG AAGTAGTATG TTATTCCTGG 2099
   TTAAGCAGGC ATTGCTTTGC CCTGGAGCAG CTATTTTAAG CCATCTCAGA TTCTGTCTAA 2159
   AGGGGTTTTT TGGGAAGACG TTTTCTTTAT CGCCCTGAGA AGATCTACCC CAGGGAGAAT 2219
   CTGAGACATC TTGCCTACTT TTCTTTATTA GCTTTCTCCT CATCCATTTC TTTTATACCT 2279
   TTCCTTTTTG GGGAGTTGTT ATGCCATGAT TTTTGGTATT TATGTAAAAG GATTATTACT 2339
   AATTCTATTT CTCTATGTTT ATTCTAGTTA AGGAAATGTT GAGGGCAAGC CACCAAATTA 2399
   CCTAGGCTGA GGTTAGAGAG ATTGGCCAGC AAAAACTGTG GGAAGATGAA CTTTGTCATT 2459
   ATGATTTCAT TATCACATGA TTATAGAAGG CTGTCTTAGT GCAAAAAACA TACTTACATT 2519
   TCAGACATAT CCAAAGGGAA TACTCACATT TTGTTAAGAA GTTGAACTAT GACTGGAGTA 2579
   AACCATGTAT TCCCTTATCT TTTACTTTTT TTCTGTGACA TTTATGTCTC ATGTAATTTG 2639
   CATTACTCTG GTGGATTGTT CTAGTACTGT ATTGGGCTTC TTCGTTAATA GATTATTTCA 2699
   TATACTATAA TTGTAAATAT TTTGATACAA ATGTTTATAA CTCTAGGGAT ATAAAAACAG 2759
   ATTCTGATTC CCTTCAAAAA AAAAAAA 2786
(2) ANGABEN ZU SEQ ID NO: 2:
   (i) SEQUENZKENNZEICHEN:
      (A) LÄNGE: 157 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Leu Val Leu Leu Ala Gly Ile Phe Val Val His Ile Ala Thr Val
    1               5                  10                  15
   Ile Met Leu Phe Val Ser Thr Ile Ala Asn Val Trp Leu Val Ser Asn
               20                  25                  30
   Thr Val Asp Ala Ser Val Gly Leu Trp Lys Asn Cys Thr Asn Ile Ser
           35                  40                  45
   Cys Ser Asp Ser Leu Ser Tyr Ala Ser Glu Asp Ala Leu Lys Thr Val
       50                  55                  60
   Gln Ala Phe Met Ile Leu Ser Ile Ile Phe Cys Val Ile Ala Leu Leu
   65                  70                  75                  80
   Val Phe Val Phe Gln Leu Phe Thr Met Glu Lys Gly Asn Arg Phe Phe
                   85                  90                  95
   Leu Ser Gly Ala Thr Thr Leu Val Cys Trp Leu Cys Ile Leu Val Gly
               100                 105                 110
   Val Ser Ile Tyr Thr Ser His Tyr Ala Asn Arg Asp Gly Thr Gln Tyr
           115                 120                 125
   His His Gly Tyr Ser Tyr Ile Leu Gly Trp Ile Cys Phe Cys Phe Ser
       130                 135                 140
   Phe Ile Ile Gly Val Leu Tyr Leu Val Leu Arg Lys Lys
   145                 150                 155
(2) ANGABEN ZU SEQ ID NO: 3:
   (i) SEQUENZKENNZEICHEN:
      (A) LÄNGE: 2614 Basenpaare
      (B) ART: Nucleotid
      (C) STRANGFORM: beides
      (D) TOPOLOGIE: linear
   (ii) ART DES MOLEKÜLS: cDNA
   (vii) UNMITTELBARE HERKUNFT:
      (B) CLON(E): Maus TMP
   (ix) MERKMAL:
      (A) NAME/SCHLÜSSEL: CDS
      (B) LAGE:90..569
   (xi) SEQUENZBESCHREIBUNG: SEQ ID NO: 3:
   GACCAGCAGA CTGCTCTACC ACCCAGGGCA TCTGCCTCTC TCACTGGATA CTCCAGAATT       60
   CTCTACTCAG AAGTCACCAA AAAGCCAAG ATG TTG GTG CTA CTG GCT GGT CTC        113
                                   Met Leu Val Leu Leu Ala Gly Leu
                                               160                 165
   TTT GTG GTC CAC ATT GCC ACT GCC ATT ATG CTG TTT GTC TCC ACC ATT        161
   Phe Val Val His Ile Ala Thr Ala Ile Met Leu Phe Val Ser Thr Ile
                   170                 175                 180
   GCC AAC GTC TGG ATG CTT GCA GAT TAC GCA AAT GCA TCT GTA GGG CTT        209
   Ala Asn Val Trp Met Val Ala Asp Tyr Ala Asn Ala Ser Val Gly Leu
               185                 190                 195
   TGG AAG AAC TGC ACT GGT GGT AAC TGC GAC GGC TCC CTG TCC TAC GGC        257
   Trp Lys Asn Cys Thr Gly Gly Asn Cys Asp Gly Ser Leu Ser Tyr Gly
           200                 205                 210
   AAT GAA GAT GCT ATC AAG GTA GTG CAA GCC TTC ATG ATC CTC TCC ATC 305
   Asn Glu Asp Ala Ile Lys Val Val Gln Ala Phe Met Ile Leu Ser Ile
   215 220 225
   ATC TTC TCC ATC ATC TCC CTC GTG GTC TTC GTG TTC CAG CTC TTC ACT 353
   Ile Phe Ser Ile Ile Ser Leu Val Val Phe Val Phe Gln Leu Phe Thr
   230 235 240 245
   ATG GAG AAG GGA AAC CGG TTC TTC CTC TCG GGG TCC ACC ATG CTG GTG 401
   Met Glu Lys Gly Asn Arg Phe Phe Leu Ser Gly Ser Thr Met Leu Val
   250 255 260
   TGC TGG CTG TGT ATC CTG GTT GGA GTG TCA ATC TAC ACT CAT CAT TAC 449
   Cys Trp Leu Cys Ile Leu Val Gly Val Ser Ile Tyr Thr His His Tyr
   265 270 275
   GCC CAC AGC GAA GGG AAC TTC AAC TCC AGC AGC CAC CAA GGC TAT TGT 497
   Ala His Ser Glu Gly Asn Phe Asn Ser Ser Ser His Gln Gly Tyr Cys
   280 285 290
   TTC ATC CTG ACC TGG ATC TGC TTC TGT TTC AGC TTC ATC ATC GGC ATA 545
   Phe Ile Leu Thr Trp Ile Cys Phe Cys Phe Ser Phe Ile Ile Gly Ile
   295 300 305
   CTC TAT ATG GTC CTG AGG AAG AAA TAAGCCGAAT ACGCTCATGG GCGTCTGGGG 599
   Leu Tyr Met Val Leu Arg Lys Lys
   310 315
   GCGGGGTGGG CTGGGTAGGA GGAAGCAACC TAACCTGGGA GGGAAGCAGG AGTCACTGTG 659
   TAGGAATAAC AGAGAGGGGA GGGGGGTGGG GAGAGGGAAG GAAGAGGGGG AGAGGCCCAA 719
   ACCCAAACCA TATCTGGGGC GGTGGGATTC TCTACTGCCA GGCACCCATC CTTGGAAGAA 779
   AGTTGTTGGC TGATATGCTG ATGCTTCCTT GACGTCACCA GAGAGTCCTC CTCTAGCCAC 839
   CGAATATGGC TCCGTCCATC CTCAATTACA TACACTCGGG GCCTCCCCAG CTGCCATACC 899
   ACTGGCGCCA CTCTTGAGGG TGGCTGCTGG GTCACACACT GAGGTCTTCC ACATCCATAT 959
   CATCAAGTTC TGATGGTGGT TCAGGTCTTA GCAAGAGCAG ATATTGCCCG ATGCTGAGGC 1019
   TAAGTCTGGA AGCCACTTTG TCCTTGTGAC CTAAAACCAA ACATCAAATC CAGATCCCAT 1079
   GTGCCTGTAG TGGGAGCTTT GGCCAGGAAG CCAATGTGCA TATTTGGGTG GCCTTTCTAA 1139
   CAAAAGTATA GGATGATGAG AGATGGTTTG TAAGTTCAAG CTGATGGAAT TGGTTTAGCC 1199
   AAGAAATGGA AGTTTCTACC CCAGAGGATC TTGGAGACAG GTGGGGACAG GCAGTGCTCC 1259
   TCAGTCACGT GTCACCGAGC TGTCCCTCAT GGAGGCCTCC TGTTGTGAAC TCTGCTAGAC 1319
   TCTCACTTAC AGCCAAGGCA GCTTTTCTGG AGTTTTTCTA GATTCTCTAG AGCCAAAGAT 1379
   GATAATGCCT CACAAAACAT AGGGTCAAAG CATATGCCCA CCGCAGTGCT ATAGTAAGTT 1439
   TGTGGGTTTT TAGGATTCCC CCAAAGCACT CAATGTATCT TGTATATGTA ACAGGGGAGA 1499
   AATGCATGTG TTCCTTTGAC ATACAATTCT GAACTAGGAA TATTTGAGGA AGTCCAATGA 1559
   TGACCAACAA CACTGGGGAC CGATAATATA ACATCTAAAT GCAGTAGTCA CTGTTGCTTT 1619
   GACCTGGGCT GGAGTGGTCT CCTCTCAACA GCTTTCATCA CACTATTTTC CAGCTAAAGA 1679
   TGGCAAAGCT GTAAGCCAAT TAACATATAC ACCAACCTAA ACTAAAGAAC CAGTCCTGAG 1739
   GGTGTGAGCA AAGGTGCTAT CTGGTTATGG ATTATTAAGC AAACCATATT TCATTTATGT 1799
   TGAGAAGAGA ATGCCTGCCC TCAGGGAAAA AAAAATGTAA TTGTGTGAGA TGAATAAAGT 1859
   CCTGGTGATA GGCAGACAGT TTCTTTTTTA AAACAGGAGA AACTCTTAGG GCATCCAGAC 1919
   AGATGGTAGC TAAATTGTTG GGGCTGCAGG GGTATTCCTG TATAAGACTT AGAGGTAGTA 1979
   TGATATCTCA GATTTCTGCC TTAAAGGGCT TTCTTTTTAG AATAGTTTCT TTTTATTGCC 2039
   CTTAGAAGAT CATTTTTAGG AAGAGTATGA GCTATCTTTT CTACAATTCT TTTCCTAGGG 2099
   AATATTCTTA TCCATTTCTT AAATACAATT CTTTTGGGAG GGAGTTTTTA TGCTATAGTT 2159
   GCTGGTATTT ATGTAAAGGG ACCCATTACT AAGTGTATTT CTCTAGCATA TTATGTTTAA 2219
   GGGACTGTTC AAGGTAGGTT ACTGAACTGC CGGGCTGATG TTAGAGACAC TGGCCAGAAA 2279
   AGCTATAATA AGTTCTTAAA TTATAATTTG ATCACTATAC ATTTGTTCTA TGCTGCCTTA 2339
   TGTTCATAAG AATATCAACA GACAGCTAGA GCCAGAAAAA AATAGTCACA TTTTGTTAAA 2399
   AAGTTGAATT ATGACTGGAG TGAACCGTGC ATTCCCTTGC CTTTTACTTT TTTTCTGTGA 2459
   CATTTACGTC TCATGTAATT TGCATCGCAT TGGTGGGTTA TTCTAGTACT GTATTTGGCT 2519
   TCTTCATTAA TAGGATATTT CACATACTAT AATTGTAAAT ATTTTGATAC AAATGTTTAT 2579
   AACTCTAGGG ATATAAAAAC AAATTCTGAT TCCCT 2614
(2) ANGABEN ZU SEQ ID NO: 4:
   (i) SEQUENZKENNZEICHEN:
      (A) LÄNGE: 160 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
   Met Leu Val Leu Leu Ala Gly Leu Phe Val Val His Ile Ala Thr Ala
    1               5                  10                  15
   Ile Met Leu Phe Val Ser Thr Ile Ala Asn Val Trp Met Val Ala Asp
               20                  25                  30
   Tyr Ala Asn Ala Ser Val Gly Leu Trp Lys Asn Cys Thr Gly Gly Asn
           35                  40                  45
   Cys Asp Gly Ser Leu Ser Tyr Gly Asn Glu Asp Ala Ile Lys Val Val
       50                  55                  60
   Gln Ala Phe Met Ile Leu Ser Ile Ile Phe Ser Ile Ile Ser Leu Val
   65                  70                  75                  80
   Val Phe Val Phe Gln Leu Phe Thr Met Glu Lys Gly Asn Arg Phe Phe
                   85                  90                  95
   Leu Ser Gly Ser Thr Met Leu Val Cys Trp Leu Cys Ile Leu Val Gly
               100                 105                 110
   Val Ser Ile Tyr Thr His His Tyr Ala His Ser Glu Gly Asn Phe Asn
           115                 120                 125
   Ser Ser Ser His Gln Gly Tyr Cys Phe Ile Leu Thr Trp Ile Cys Phe
       130                 135                 140
   Cys Phe Ser Phe Ile Ile Gly Ile Leu Tyr Met Val Leu Arg Lys Lys
   145 150 155 160
(2) ANGABEN ZU SEQ ID NO: 5:
   (i) SEQUENZKENNZEICHEN:
      (A) LÄNGE: 756 Basenpaare
      (B) ART: Nucleotid
      (C) STRANGFORM: beides
      (D) TOPOLOGIE: linear
   (ii) ART DES MOLEKÜLS: cDNA
   (vii) UNMITTELBARE HERKUNFT:
      (B) CLON(E): Human TMP
   (ix) MERKMAL:
      (A) NAME/SCHLÜSSEL: CDS
      (B) LAGE:1..471
   (xi) SEQUENZBESCHREIBUNG: SEQ ID NO: 5:
   ATG TTG GTG CTA CTG GCT GGT ATC TAT GTG GTC CAC ATC GCT ACT GTT 48
   Met Leu Val Leu Leu Ala Gly Ile Tyr Val Val His Ile Ala Thr Val
   165 170 175
   ATT ATG CTA TTT GTT AGC ACC ATT GCC AAT GTC TGG TTG GTT TCC AGT 96
   Ile Met Leu Phe Val Ser Thr Ile Ala Asn Val Trp Leu Val Ser Ser
   180 185 190
   ACG GCA GAT GCA TCA GTA GGT CTT TGG AAA AAC TGT TCC AAC ATG GAG 144
   Thr Ala Asp Ala Ser Val Gly Leu Trp Lys Asn Cys Ser Asn Met Glu
   195 200 205
   TGC AGT GAC AGC CTG TCA TAT GCC AGT GAA GAT GCC CTC AAG ACA GTG 192
   Cys Ser Asp Ser Leu Ser Tyr Ala Ser Glu Asp Ala Leu Lys Thr Val
   210 215 220
   CAG GCC TTC ATG ATT CTC TCT ATC ATC TTC TGT GTC ATT GCC CTC CTG          240
   Gln Ala Phe Met Ile Leu Ser Ile Ile Phe Cys Val Ile Ala Leu Leu
   225                 230                 235                 240
   GTC TTC GCG TTC CAG CTC TTC ACC ATG GAG AAG GGA AAC CGG TTC TTC          288
   Val Phe Ala Phe Gln Leu Phe Thr Met Glu Lys Gly Asn Arg Phe Phe
                   245                 250                 255
   CTC TCA GGG GCC ACC ACA CTG GTG TGC TGG CTG TGC ATT CTT GTG GGG          336
   Leu Ser Gly Ala Thr Thr Leu Val Cys Trp Leu Cys Ile Leu Val Gly
               260                 265                 270
   GTG TCC ATC TAC ACT AGT CAT TAT GCG AAT CGT GAT GGA ACG CAG TAT          384
   Val Ser Ile Tyr Thr Ser His Tyr Ala Asn Arg Asp Gly Thr Gln Tyr
           275                 280                 285
   CAC CAC GGC TAT TCC TAC ATC CTG GGC TGG ATC TGC TTC TGC TTC AGC          432
   His His Gly Tyr Ser Tyr Ile Leu Gly Trp Ile Cys Phe Cys Phe Ser
       290                 295                 300
   TTC ATC ATC GGC GTT CTC TAT CTG GTC CTG AGA AAG AAA TAAGGCCGGA           481
   Phe Ile Ile Gly Val Leu Tyr Leu Val Leu Arg Lys Lys
   305                 310                 315
   CGAGTTCATG GGGATCTGGG GGGTGGGGAG GAGGAAGCCG TTGAATCTGG GAGGGAAGTG        541
   GAGGTTGCTG TACAGGAAAA ACCGAGATAG GGGAGGGGGG AGGGGGAAGC AAAGGGGGGA        601
   GGTCAAATCC CAAACCATTA CTGAGGGGAT TCTCTACTGC CAAGCCCCTG CCCTGGGGAG        661
   AAAGTAGTTG GCTAGTACTT TGATGCTCCC TTGATGGGGT CCAGAGAGCC TCCCTGCAGC        721
   CACCAGACTT GGCCTCCAGC TGTTCTTAGT GACAC                                   756
(2) ANGABEN ZU SEQ ID NO: 6:
   (i) SEQUENZKENNZEICHEN:
      (A) LÄNGE: 157 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Leu Val Leu Leu Ala Gly Ile Tyr Val Val His Ile Ala Thr Val
   1 5 10 15
   Ile Met Leu Phe Val Ser Thr Ile Ala Asn Val Trp Leu Val Ser Ser
   20 25 30
   Thr Ala Asp Ala Ser Val Gly Leu Trp Lys Asn Cys Ser Asn Met Glu
   35 40 45
   Cys Ser Asp Ser Leu Ser Tyr Ala Ser Glu Asp Ala Leu Lys Thr Val
   50 55 60
   Gln Ala Phe Met Ile Leu Ser Ile Ile Phe Cys Val Ile Ala Leu Leu
   65 70 75 80
   Val Phe Ala Phe Gln Leu Phe Thr Met Glu Lys Gly Asn Arg Phe Phe
   85 90 95
   Leu Ser Gly Ala Thr Thr Leu Val Cys Trp Leu Cys Ile Leu Val Gly
   100 105 110
   Val Ser Ile Tyr Thr Ser His Tyr Ala Asn Arg Asp Gly Thr Gln Tyr
   115 120 125
   His His Gly Tyr Ser Tyr Ile Leu Gly Trp Ile Cys Phe Cys Phe Ser
   130 135 140
   Phe Ile Ile Gly Val Leu Tyr Leu Val Leu Arg Lys Lys
   145 150 155